# EUROPEAN PATENT SPECIFICATION

(11) **EP 1 246 594 B1**
(45) Date of publication and mention of the grant of the patent: **26.05.2004**
(21) Application number: 00956043.4
(22) Date of filing: 08.09.2000
(51) Int. Cl.: A61F 13/514, A61F 13/84

(54) **NONWOVEN COVERSTOCK FOR ABSORBENT ARTICLES**
VLIESSTOFFDECKLAGE FÜR ABSORBIERENDE ARTIKEL
GARNITURE NON TISSEE POUR ARTICLES ABSORBANTS

(30) Priority: 17.09.1999 CZ 332799
(43) Date of publication of application: 09.10.2002
(73) Proprietor: Pegas A.S., 669 04 Znojmo (CZ)
(72) Inventor: KLASKA, Frantisek, 684 01 Slavkov u Brna (CZ); RUZ, Ivo, Edward, D-67663 Kaiserslautern (DE)
(74) Representative: Gallafent, Richard John
(86) International application number: PCT/CZ2000/000063
(87) International publication number: WO 2001/021125

(56) References cited:
- EP-A- 0 619 393
- EP-A- 0 640 329
- WO-A-98/22056
- US-A- 4 868 031
- US-A- 5 763 334

## Description

The invention provides a soft hydrophobic nonwoven coverstock for absorption hygienic items made of continuous filaments i.e. a spunlaid nonwoven fabric with a titre of less than 1.5 dtex and containing 0.00 to 0.15 % titanium dioxide as a dulling agent. The most advantageous concentration of titanium dioxide is between 0.01 to 0.12 %. The invention provides further such nonwoven coverstock consisting of more than 65 % polypropylene.

### Background of the invention

Hydrophilic and permeable coverstock materials of a similar kind are well known and are used for manufacturing hygienic absorption items such as baby diapers, diapers for incontinent adults and similar items. Such items consist of an absorption core made of wood pulp and containing granulated SAP (superabsorption polymer). The backside of such absorption items which is opposite to the user's body is made of a nonpermeable sheet, mostly of a polymer film which eventually can be laminated with a nonwoven fabric. The inner face of such items, which is in contact with the user's body, will be made of a permeable material, mostly a permeable nonwoven fabric. In order to limit the reverse penetration of the absorbed fluid, it is useful to use a permeable coverstock over only a central strip and not over the entire area of the item. The rest of the surface, or the side panels, can be made of a soft textile material which is hydrophobic. The same hydrophobic material can be used to form barrier leg cuffs providing additional sealing and hindering leakage of the fluid (urine) outside of the item.

State-of-the-art composite multiply nonwoven fabrics may be used as barrier leg cuffs. Typical composite fabrics of this kind consist of two nonwoven plies made of continuous filaments of a filament denier 1.5 dtex to 3.0 dtex with a smooth ply made of hyperfine fibrils called "meltblown". Such laminates would typically provide a standard weight of 17 to 20 g/m², where the central meltblown ply provides 15 to 25 % of the total weight. The most important criterion for such composite barrier leg cuff materials called SMS (to reflect their spunlaid/meltblown/spunlaid structure) is their level of hydrophobia expressed as waterhead which has to exceed 100 mm. The hydrophobia of this composite material is governed by the meltblown ply. The contribution of the regular fibres is rather limited, but they are important as constituents responsible for the strength properties.

A SMS composite as described is opaque due to the optical properties of the meltblown microfibrils. In addition, the basic nonwoven is regularly dulled by adding 0.4 % titanium dioxide or more.

It is known to apply cosmetic lotion at the coverstock of hygienic items for improved skin protection of the user. Such treatment is described in DE-PS 35 36 318 as well as in DE-PS 35 36 319. When a simple nonwoven fabric is used in combination with the treatment, as described in the patents mentioned above, no particular optical problems occur. This is due to the fact that simple nonwoven coverstock, even if made of dull filaments containing regularly about 0.4 % titanium dioxide, is not particularly opaque.

By contrast, when cosmetic lotions, which are normally water dispersions containing oil and/or fat, are applied to a highly opaque nonwoven sheet, non regular oil or fat spots can occur even when uniform application is provided very carefully. Such spots are very detrimental to the general optical impression. Thereby such spots are not necessarily due to quantitative irregularities of the application. Rather they are optical phenomena based on differences in the surface wetting of fibres.

It is known to use composite nonwoven fabrics containing super micro fibres, known as melt blown, for hydrophobic parts of hygienic devices such as diapers - see for example WO-A-9822056. Such composites have two, or preferably three plies, and are always a combination of one or two plies respectively made of regular fibres or filaments and at least one ply made of said melt blown super micro fibres. The high degree of hydrophobia is achieved by the fact that the ply containing melt blown super micro fibres provides the hydrophobic properties due to very non-favourable angles between individual fibres. A web made of such super micro fibres cannot be wetted and penetrated by water. Nevertheless, the melt blown super micro fibres provide a non-acceptably high degree of opacity so that composite webs containing them are opaque, which is the main disadvantage of such composite webs.

It is known - see US Patent 5,763,334 or EP-A-0619393 - to make carded nonwovens with improved hydrophobia from polyolefin staple fibres of various titres containing internal hydrophobic polysiloxane of a given particular formula - the polyolefin staple fibres are modified by the polysiloxanes. These staple fibres improve the hydrophobia of such carded nonwoven webs and make them suitable for use in hygienic devices such as diapers. Such devices are improved by these fibres, which, as spun, present a greater hydrophobia than regular known polyolefin fibres lacking the internal siloxane lubricant. The improved hydrophobia is evidenced by an advancing contact angle for the as spun fibres of at least about 95°.

It has been suprisingly demonstrated, that nonwovens made of spunlaid continuous filaments of a titre of less than 1.5 dtex and particularly those of more than 0.01 and less than 1.2 dtex, are capable of reaching a high level of hydrophobia with waterhead figures of more than 100 mm, due to their geometry and non favourable wetting angles. This enables the use of such nonwovens as a breathable barrier fabric for hygienic items without needing to employ any meltblown ply.

It has been further surprisingly demonstrated, that nonwoven fabrics made of microfibres keep their favourable surface properties even when made with a very low proportion of titanium dioxide below 0.15 % or without any titanium dioxide at all. However the preferred proportion of titanium dioxide is in a range between 0.01 and 0.12 %, a range which improves the spinning and bonding processes.

It has been further demonstrated, that nonwoven fabrics according to this invention can not only be made of simple fibres but in similar way of bi-component fibres or of a fibre mix of two different kinds of fibres. It is possible to use another polyolefin as a second constituent, e. g. polyethylene, a random co-polymer of polypropylene or another spinnable polymer, such as polyester.

### Example 1

A spinning machine for nonwoven fabrics of type *Reicofil*®-3 (made by the Reifenhäuser Company) consisting of two spinning beams was adjusted as necessary for manufacturing of microfilaments. The polypropylene *Metocene*® with a MFR 30 manufactured by the Targor AG Company has been used as a raw material.

Both spinning beams used the same spinning conditions as follows:

At a spinning beam width of 3.2 m was installed a spinneret with 17700 orifices. The melt temperature was 245-250 °C, the temperature of the spinneret 250-255 °C. The total throughput of polymer was 5.8 kg/min, i.e. 0.33 g/min per orifice. The dosage of titanium dioxide masterbatch was installed so that the polymer melt as spun contained 0.1 % of the pigment. Continuous filaments extruded into the quenching and drawing cabinet were quenched by air of a temperature of 25 °C, which had been accelerated in the drawing part of the cabinet and used for drawing. The width of the narrow part of the cabinet was 17 mm. The bottom part of the cabinet broadened and formed a diffuser where the drawn continuous filaments were laid up at a collector screen in the form of a fibrous sheet whereby this web forming section of the collector screen was equipped from the bottom side with a suction cabinet. The collector screen transported the fibrous sheet to a hot calender for bonding. The screen speed was adjusted at 200 m/min so, that the weight of the nonwoven fabric was 17 g/m². The calender bonding temperature was 155 °C at the patterned cylinder and 143 °C at the smooth one.

The manufactured spunlaid nonwoven provided the following laboratory test results (MD stands for machine direction , CD for cross machine direction ):

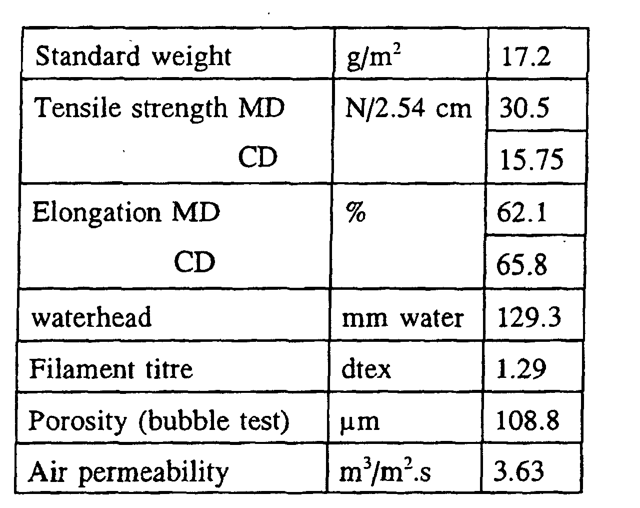

The spunlaid nonwoven fabric manufactured in trial was almost transparent and when a fat or oil dispersion was applied no spoilage was observed. Such nonwoven fabric is very suitable as a barrier coverstock for use in the manufacturing of baby diapers.

### Example 2

In this case the same two spinning beam manufacturing line as described in Example 1 was used. In the same way, the spinning conditions corresponded exactly to what has been described in Example 1, except that the throughput was increased up to 7.0 kg/min or 0.40 g/min per orifice respectively. No titanium dioxide as pigment was added. To keep the basic weight of the manufactured web unchanged, the speed of the collector screen was increased to 234 m/min. The manufactured spunlaid nonwoven fabric provided the following laboratory test results:

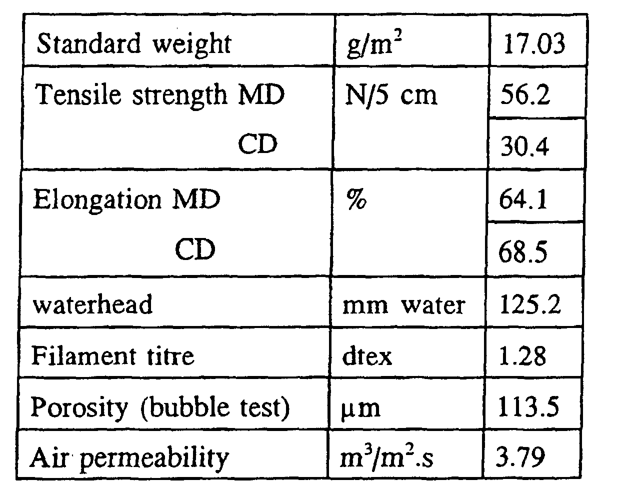

The nonwoven spunlaid web manufactured according to this example was fully transparent and it allowed a treatment with a fat or oil dispersion without any visible spoilage. Such a nonwoven material is very suitable as a hydrophobic barrier fabric for manufacturing baby diapers.

### Comparative example 1

A spunlaid nonwoven web was manufactured according to Example 2, the only difference was the addition of 0.5 % titanium dioxide masterbatch. The test results of this material were exactly according to the table in Example 2.

The manufactured web was quite opaque, which was the main difference to Example 2. After application of an oil dispersion poorly defined spots were observed which resulted in a non-favourable visual impression. The possibility for use of such a material as a barrier fabric for baby diapers is quite limited even if all laboratory tests provided excellent results.

### Comparative example 2

The same machine as described in Example 1 was used, except that a meltblown spinning beam was installed between both spinning beams for continuous filaments. In this way a composite SMS material was manufactured consisting of a meltblown ply sandwiched between two spunlaid plies. Out of the total basic weight of 17 g/m², the constituent spunlaid plies each provided 7 g/m² with 3 g/m² meltblown in-between. The spunlaid materials provided filament titre of 1.2 dtex, the meltblown fibrils were as usual quite irregular with an average of 0.4 dtex.

The following are the results of laboratory tests:

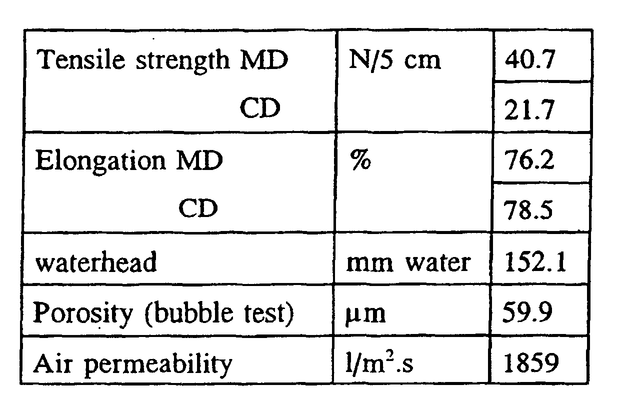

The composite SMS material manufactured according to this example provides an excellent fluid barrier. Unfortunately, it is highly opaque and even when treated very carefully with an oil dispersion there is a strong tendency to form spots detrimental to the visual impression.

## Claims

1. Hydrophobic nonwoven coverstock for absorption hygienic items, **characterized in that** it is made of spunlaid continuous filaments of a titre of less than 1.5 dtex and contains less than 0.15 % titanium dioxide as dulling agent.

2. Hydrophobic nonwoven coverstock according to claim 1, **characterized in that** the filaments have a titre of less than 1.2 dtex.

3. Hydrophobic nonwoven coverstock according to claim 1 or 2, **characterized in that** the filaments contain 0.01 to 0.12 % titanium dioxide.

4. Hydrophobic nonwoven coverstock according to any one of claims 1 to 3, **characterized in that** the proportion of polypropylene in the filaments is higher than 65 %.

## Patentansprüche

1. Hydrophober Aladeckvliesstoff für hygienische Absorbtionsartikel **dadurch gekennzeichnet, dass** es aus direkt ersponnenen Endlosfäden, die einen Titer von weniger als 1,5 dtex besitzen und die weniger als 0,15 % Titandioxid als Mattierungsmittel enthalten, hergestellt wird.

2. Hydrophober Abdeckvliesstoff nach Anspruch 1 **dadurch gekennzeichnet, dass** die Endlosfäden einen Titer von weniger als 1,2 dtex besitzen.

3. Hydrophober Abdeckvliesstoff nach Anspruch 1 und 2 **dadurch gekennzeichnet, dass** die Endlosfäden 0,01 bis 0,12 % Titandioxid enthalten.

4. Hydrophober Abdeckvliesstoff nach einem der Ansprüche 1 bis 3 **dadurch gekennzeichnet, dass** der Anteil von Polypropylen in den Endlosfäden höher als 65 % ist.

## Revendications

1. Garniture non-tissée hydrophobe pour des articles d'hygiène absorbants, **caractérisée en ce qu'**elle est faite de filaments continus désorientés d'un titre inférieur à 1,5 dtex et qu'elle contient moins de 0,15 % de dioxyde de titane en tant qu'agent matifiant.

2. Garniture non-tissée hydrophobe selon la revendication 1, **caractérisée en ce que** les filaments ont un titre inférieur à 1,2 dtex.

3. Garniture non-tissée hydrophobe selon la revendication 1 ou 2, **caractérisée en ce que** les filaments contiennent 0,01 à 0,12 % de dioxyde de titane.

4. Garniture non-tissée hydrophobe selon l'une quelconque des revendications 1 à 3, **caractérisée en ce que** la proportion de polypropylène dans les filaments est supérieure à 65 %.
